Europäisches Patentamt

European Patent Office

Office européen des brevets

(11) Numéro de publication: **0 345 138**
**A1**

## DEMANDE DE BREVET EUROPEEN

(21) Numéro de dépôt: **89401459.6**

(22) Date de dépôt: **29.05.89**

(51) Int. Cl.⁴: **A 61 B 6/00**

(30) Priorité: **03.06.88 FR 8807442**

(43) Date de publication de la demande:
**06.12.89 Bulletin 89/49**

(84) Etats contractants désignés: **DE ES GB IT NL**

(71) Demandeur: **GENERAL ELECTRIC CGR S.A.**
**100, rue Camille-Desmoulins**
**F-92130 Issy les Moulineaux (FR)**

(72) Inventeur: **Lajus, Pierre CABINET BALLOT-SCHMIT**
**84, avenue Kléber**
**F-75116 Paris (FR)**

**Caugant, Jean CABINET BALLOT-SCHMIT**
**84, avenue Kléber**
**F-75116 Paris (FR)**

(74) Mandataire: **Ballot, Paul Denis Jacques**
**Cabinet Ballot-Schmit 7, rue le Sueur**
**F-75116 Paris (FR)**

(54) **Statif de radiologie isocentrique à quatre axes de rotation.**

(57) L'invention concerne des statifs de radiologie du type à mouvements d'exploration isocentrique.

L'invention réside dans le fait que dans un statif à trois axes de rotation (1, 2 et 3) concourants à l'isocentre O, il est prévu de faire tourner l'isocentre autour d'un quatrième axe (Axe 4) perpendiculaire au deuxième axe (Axe 2) et parallèle au troisième axe (Axe 3). Ceci permet de déplacer l'isocentre (O) dans le plan horizontal le contenant, notamment suivant des droites sécantes au troisième axe.

L'invention est applicable aux installations de radiologie.

FIG_1

EP 0 345 138 A1

## Description

### STATIF DE RADIOLOGIE ISOCENTRIQUE A QUATRE AXES DE ROTATION

L'invention concerne les installations de radiologie et, plus particulièrement dans une telle installation, un statif de radiologie qui permet une exploration ou examen isocentrique d'un patient sous des incidences multiples.

L'examen radiologique d'un patient s'effectue à l'aide d'une chaîne image qui, généralement, est constituée essentiellement d'une source de rayons X, d'un collimateur, d'une grille anti-diffusante et d'un récepteur qui sont portés et liés rigidement entre eux par une structure appelée statif. La source de rayons X et le collimateur sont d'un même côté par rapport au patient à examiner qui est disposé sur une table tandis que la grille anti-diffusante et le récepteur sont du côté opposé. Une droite passant par le foyer de la source de rayons X et le centre du récepteur représente l'axe du rayonnement X ou axe de la chaîne image. Dans le cas d'une exploration ou examen isocentrique, cet axe passe toujours en un même point d'une zone à analyser quelle que soit l'orientation dudit axe, ce point constituant l'isocentre. Le mouvement qui permet de faire varier l'orientation de l'axe de la chaîne image par rapport à l'isocentre est appelé mouvement isocentrique.

Les statifs qui permettent de réaliser un mouvement isocentrique comportent généralement un arceau ouvert dont une extrémité porte la source de rayons X et l'autre extrémité porte le récepteur. L'axe de la chaîne image passe par l'isocentre qui constitue le centre de l'arceau ou est sur un même axe que le centre de l'arceau de sorte qu'un premier mouvement isocentrique est réalisé en faisant tourner l'arceau sur lui-même dans son plan, autour de son centre, par exemple en faisant coulisser l'arceau dans un fourreau en forme d'arc de cercle.

Les statifs de radiologie permettent en outre un deuxième mouvement isocentrique qui consiste en une rotation du plan de l'arceau autour d'un second axe de rotation perpendiculaire au premier et passant également par l'isocentre.

Dans certains statifs de radiologie, il est prévu d'effectuer un troisième mouvement isocentrique qui consiste en une rotation autour d'un troisième axe de rotation perpendiculaire au plan des premier et second axes et passant par l'isocentre.

On comprend que ces trois mouvements isocentriques permettent de réaliser des vues en incidence du patient suivant des plans qui peuvent être orientés dans toutes les directions de l'espace.

Dans une installation de radiologie, le patient est disposé sur une table qui permet des déplacements du patient dans trois directions, l'une en élévation (axe des z) et deux dans le plan horizontal (axe des x dans la direction longitudinale du patient et axe des y dans la direction perpendiculaire). Ces mouvements ont pour but de faire coïncider l'isocentre avec le centre de la zone à examiner.

Une telle installation présente un certain nombre d'inconvénients. L'un d'entre eux est d'être encombrante car elle nécessite l'utilisation d'une table de support de patient qui se déplace dans le plan horizontal de manière à placer le centre de la zone à observer à l'isocentre du statif.

Un autre inconvénient est que le statif présente un porte-à-faux important car il doit permettre le déplacement longitudinal du patient sur une grande largeur. Ceci contribue également à augmenter l'encombrement de l'installation.

Un but de la présente invention est donc de réaliser un statif de radiologie isocentrique qui permet de réaliser des vues en incidence du patient sans avoir à déplacer ce dernier pour faire coïncider l'isocentre avec le centre de la zone à examiner.

Un autre but de la présente invention est également de réaliser un statif de radiologie isocentrique qui permet de réduire l'encombrement de l'installation de radiologie.

L'invention se rapporte à un statif de radiologie isocentrique qui comprend une source de rayons X et un détecteur de rayons X portés par un arceau et définissant un axe de chaîne image passant par un isocentre, ledit arceau étant mobile en rotation autour d'un premier axe (Axe 1) perpendiculaire au plan de l'arceau et passant par l'isocentre ainsi qu'autour d'un deuxième axe (Axe 2) dans le plan de l'arceau perpendiculaire au premier axe et passant également par l'isocentre, ledit arceau étant supporté par une structure rigide qui pivote autour d'un troisième axe (Axe 3) perpendiculaire aux premier et deuxième axes et passant par l'isocentre, caractérisé en ce qu'il comprend des moyens de rotation pour faire tourner l'isocentre autour d'un quatrième axe (Axe 4) perpendiculaire au deuxième axe et parallèle au troisième axe.

D'autres caractéristiques et avantages de la présente invention apparaîtront à la lecture de la description suivante d'un exemple particulier de réalisation, ladite description étant faite en relation avec les dessins joints dans lesquels :

- la figure 1 est une vue qui montre de manière schématique les positions relatives des différents axes de rotation d'un statif selon l'invention,

- la figure 2 est un dessin géométrique montrant le déplacement rectiligne que l'on peut obtenir à l'aide de deux mouvements angulaires coordonnés,

- la figure 3 est une vue en élévation d'un exemple de réalisation d'un statif selon l'invention,

- la figure 4 est une vue en perspective cavalière d'une installation de radiologie utilisant un statif isocentrique selon l'invention.

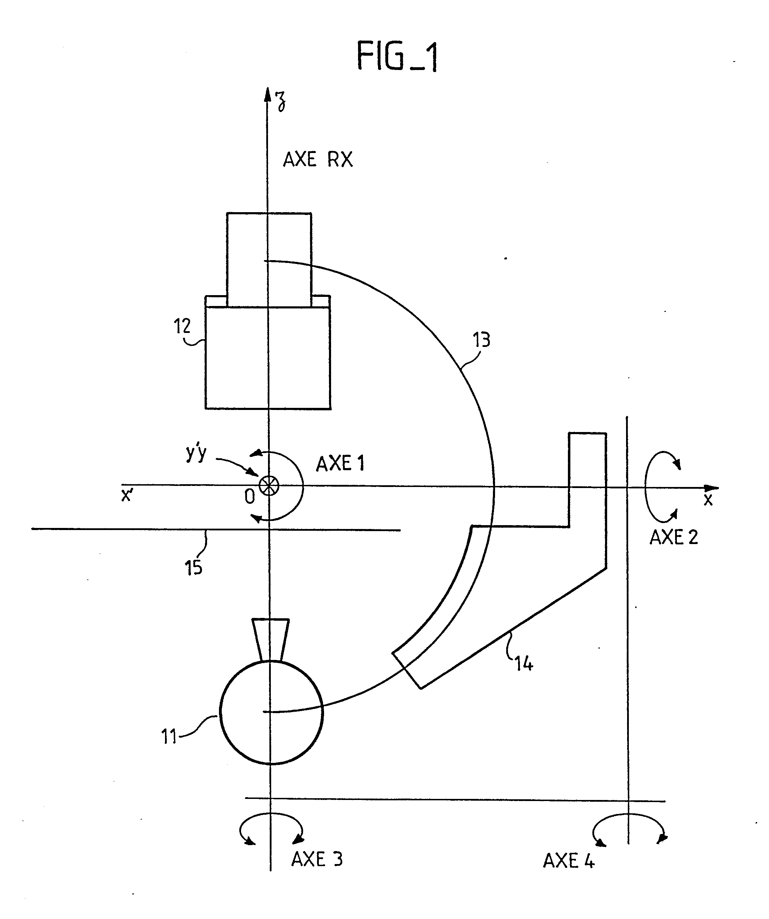

Sur la figure 1, qui est une vue montrant de manière schématique les positions relatives des différents axes de rotation d'un statif selon l'invention, on a représenté les trois axes orthogonaux x'x et y'y dans le plan horizontal et z'z dans le plan vertical qui concourent en un point O. Le statif comprend un arceau 13 qui supporte une source de rayons X 11 et un détecteur 12 disposés suivant le diamètre de l'arceau. Il est supporté par un fourreau ou glissière 14 dans lequel il peut coulisser sous la commande de l'opérateur. Une table de support de patient 15 dite table d'examen est disposée entre la source de rayons X 11 et le détecteur 12 dans le sens longitudinal x'x et légèrement au dessous du plan horizontal défini par les axes x x' et y'y.

Le coulissement de l'arceau 13 permet de réaliser une rotation de la source de rayons X et du détecteur autour d'un axe 1 perpendiculaire au plan contenant ces deux éléments, c'est-à-dire que l'axe des rayons X décrit une surface dans un plan parallèle à ce plan. Dans le cas particulier de la figure 1, l'Axe 1 est confondu avec l'axe y'y.

Le fourreau 14 peut tourner autour d'un axe 2 situé dans un plan horizontal passant par le point O appelé isocentre. Dans ce cas particulier de la figure 1, cet Axe 2 coïncide avec l'axe x'x. La rotation de l'arceau 13 autour de l'Axe 2 permet de faire décrire à l'axe des rayons X une surface perpendiculaire au plan de l'arceau.

Le support de l'Axe 2 (non représenté) peut tourner autour d'un axe 3 confondu avec l'axe vertical z'z et passant donc par l'isocentre O. Cette rotation autour de l'Axe 3 permet de déplacer l'arceau 13 d'un côté et de l'autre de la table d'examen, c'est-à-dire dégager l'accès à la tête du patient.

Ces trois rotations autour des Axes 1, 2, et 3 permettent d'effectuer des explorations de zones du patient suivant des incidences qui ont des directions quelconques dans l'espace mais qui passent toutes par l'isocentre O. On comprend alors que pour examiner un autre organe du patient, il faille déplacer ce dernier c'est-à-dire la table d'examen, pour amener le centre de cet autre organe à l'isocentre O.

Selon l'invention, il est proposé de déplacer l'isocentre O de manière à pouvoir le faire coïncider avec le centre de la zone du patient à observer en prévoyant une rotation de l'Axe 2 autour d'un axe 4 qui est perpendiculaire à l'Axe 2 et situé dans le plan contenant l'Axe 3 dans le cas de la position particulière de la figure 1. Cette rotation autour de l'Axe 4 permet de déplacer l'isocentre O dans le plan horizontal défini par les axes x'x et y'y en lui faisant décrire un arc de cercle. Comme par ailleurs l'axe 4 peut tourner autour de l'Axe 3, il est possible de faire décrire à l'isocentre O une courbe quelconque dans le plan horizontal en combinant les deux mouvements angulaires autour des Axes 3 et 4.

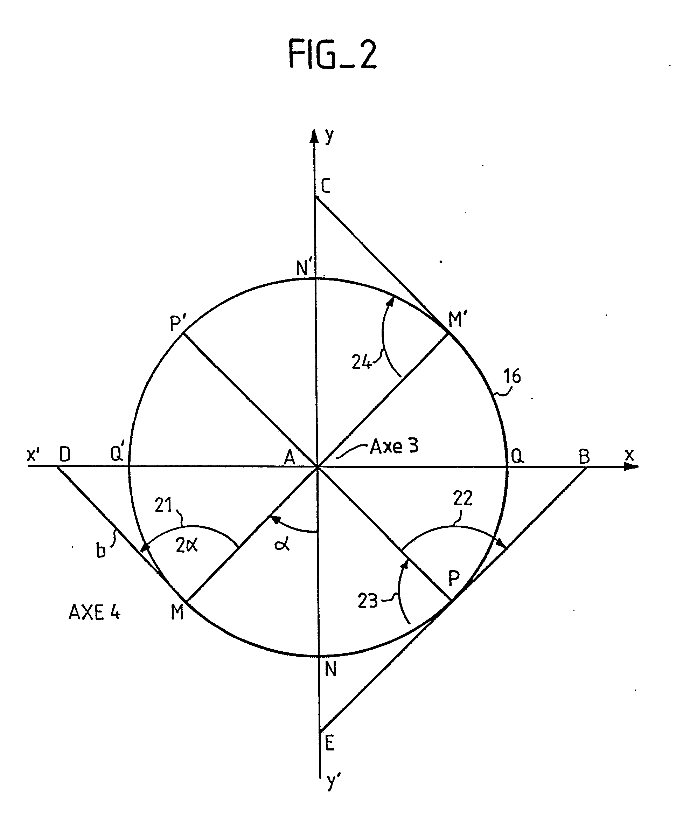

Le dessin géométrique de la figure 2 permet de comprendre les mouvements qui peuvent être effectués grâce à la présence de cet Axe 4. Ce dessin a été tracé dans le plan horizontal et le point A matérialise la position de l'isocentre ainsi que celle de l'axe z'z ou Axe 3. L'Axe 4 peut donc se déplacer sur un cercle 16 de centre A pour couvrir environ un angle de 135° de part et d'autre de l'axe x'x en passant du côté de la tête du patient. La limitation à 135° environ de part et d'autre est due à la présence du support de la table d'examen, côté des pieds du patient. Pour chaque position sur ce cercle 16, le statif a trois axes concourants ce qui permet toutes les incidences autour de l'isocentre.

La figure 2 permet également de comprendre que la combinaison du mouvement angulaire autour de l'Axe 3 et du mouvement angulaire de l'Axe 2 autour de l'Axe 4 permet de faire décrire à l'isocentre le segment DB sur l'axe x'x et le segment EC sur l'axe y'y. La loi qui relie les deux mouvements angulaires doit être telle que lorsque l'Axe 4 tourne d'un angle $\alpha$ autour de l'Axe 3, l'Axe 2 doit tourner d'un angle $2\alpha$ autour de l'Axe 4. Ainsi, lorsque l'Axe 4 est en position N, c'est-à-dire sur l'axe y'y, l'isocentre est au point A. Si l'Axe 4 tourne de l'angle $\alpha$ pour aller en position M, l'isocentre sera sur x'x au point D si l'Axe 2 tourne d'un angle $2\alpha$ autour de l'Axe 4 dans le sens 4 défini par la flèche 21. En effet, le triangle défini par les points A, M, et D doit toujours être isocèle de côté b égal à l'entraxe du statif, c'est-à-dire la distance entre l'isocentre et l'Axe 4. Il en résulte alors que l'angle au sommet doit être égal à $2\alpha$ si l'angle MAN est égal à $\alpha$.

Lorsque l'Axe 4 passe de N en P, l'isocentre décrira le segment AB si l'Axe 2 tourne dans le sens défini par la flèche 22. On comprend que l'isocentre peut décrire les segments AB et AD lorsque l'Axe 4 décrit respectivement les arcs de cercle N'M' et N'P'.

Pour que l'isocentre décrive le segment EA, l'Axe 4 doit décrire l'arc de cercle PQ tandis que l'Axe 2 doit tourner dans le sens indiqué par la flèche 23. Pour AC, l'Axe 4 doit décrire l'arc de cercle QM' tandis que l'Axe 2 doit tourner dans le sens de la flèche 24 . Ici également on comprend que l'isocentre peut décrire les segments AE et AC lorsque l'Axe 4 décrit les axes de cercle Q'M et Q'P' mais cette possibilité n'est pas utilisée car le support de la table d'examen empêche le déplacement sur une partie de l'arc de cercle P'Q'M. La longueur des segments décrits par l'isocentre sur les axes x'x et y'y est donnée par la formule

$$2b \sin \alpha$$

déterminée à l'aide des relations trigonométriques du triangle isocèle DMA par exemple.

Ce qui vient d'être démontré pour le déplacement de l'isocentre sur les axes x'x et y'y peut être démontré pour tout autre système d'axes orthogonaux incliné par rapport aux axes x'x/y'y. Ceci montre que l'isocentre peut être déplacé en tout point du plan horizontal défini par x'x et y'y mais à l'intérieur d'un cercle de rayon $2b \sin \alpha$ et de centre A.

Il est à remarquer que le rayon de ce cercle est limité par la valeur maximale que peut prendre l'angle $\alpha$ compte tenu de la présence de la table d'examen.

Lorsque l'isocentre décrit les segments AD, AB, AE et AC, le faisceau de rayons X suit le mouvement angulaire autour de l'Axe 3 et tourne d'un angle $\alpha$. Il en résulte que l'image reçue par le détecteur tourne également et qu'il faut donc effectuer des corrections pour qu'elle garde la même orientation dans l'espace. Ces corrections peuvent être obtenues par des moyens et/ou procédés électroniques en agissant sur l'image elle-même ou des moyens agissant directement sur l'orientation ou du détecteur en fonction de la valeur de l'angle $\alpha$.

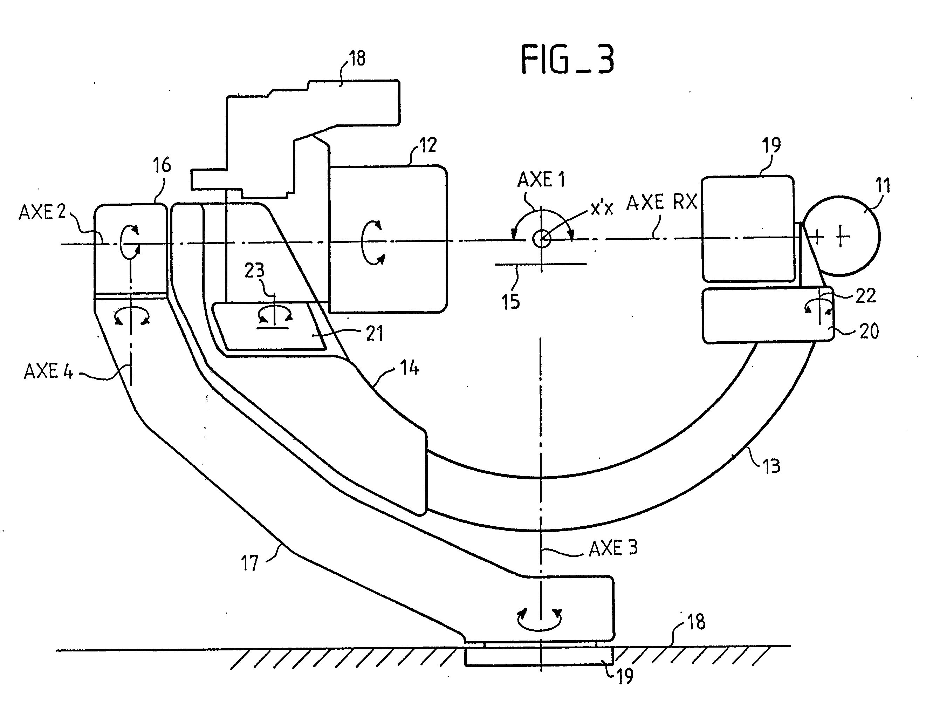

La figure 3 est une vue en élévation d'un exemple de réalisation d'un statif mettant en oeuvre l'invention décrite en relation avec les figures 1 et 2. Sur cette figure 3, les éléments identiques à ceux de la figure 1 portent les mêmes références. Le fourreau 14 est porté par un élément 16 qui porte l'arbre de rotation matérialisant l'Axe 2. Cet arbre de rotation peut tourner de plus ou moins 180°, ce qui signifie que l'arceau peut effectuer un tour complet autour de l'Axe 2. L'élément 16 est porté à une extrémité supérieure d'une structure rigide 17 en forme d'arc de cercle dont l'autre extrémité inférieure pivote autour de l'Axe 3 entre - 135° et + 135°. Cet Axe 3 est matérialisé par un arbre qui est fixé au sol 18 par un socle 19. Conformément à l'invention, l'élément 16 est monté pivotant autour de l'Axe 4 qui est monté verticalement à l'extrémité supérieure de la structure pivotante 17. Le mouvement angulaire est limité entre les positions - 90° et + 90°.

Le détecteur 12 est monté pivotant de manière à tourner autour de l'Axe des rayons X. Ceci permet de garder pour l'image la même orientation quels que soient les mouvements angulaires autour des Axes 3 et 4.

Le statif qui vient d'être décrit peut être associé avec des éléments qui sont utilisés dans les statifs de l'art antérieur. C'est ainsi que l'on peut associer à la source de rayons X une collimation 19 et au détecteur 12 de rayons X une chaîne image 18. On peut également monter la source de rayons X et le détecteur sur des dispositifs à coulissement 20 et 21, respectivement, de manière à réaliser leur déplacement relatif sur l'axe RX.

On peut également monter la source de rayons X et le détecteur sur des axes 22 et 23 respectivement, afin de travailler en rayons incidents sur le détecteur ou sur un détecteur annexe. Il est possible également de déplacer verticalement l'axe 2 de manière à obtenir une position variable de l'isocentre en hauteur.

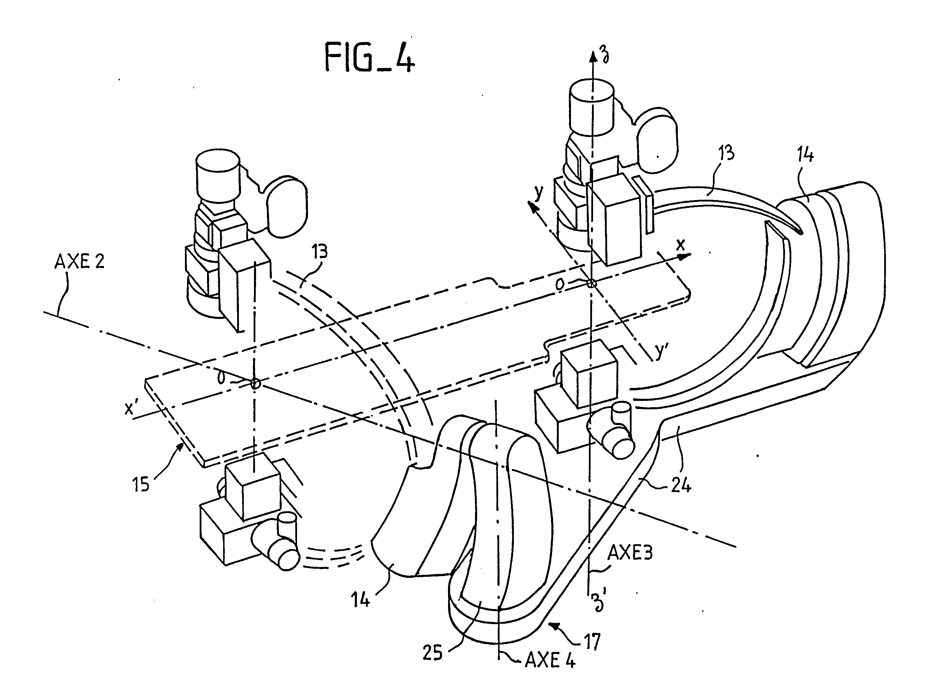

La figure 4 est un schéma en perspective cavalière d'un autre exemple de réalisation d'un statif selon l'invention dans lequel le pivot de l'Axe 4 est au niveau du sol et non pas en élévation comme c'est le cas du statif de la figure 3. Celà revient à avoir un bras 17 qui repose et se déplace sur le sol ce qui évite d'avoir un bras 17 en porte-à-faux. Sur cette figure, les éléments identiques à ceux des figures précédentes portent les mêmes références.

La figure 4 montre deux positions du statif, l'une dans laquelle l'isocentre O est au centre sur la tête du patient et l'autre dans laquelle l'isocentre O a été amené au niveau des membres inférieurs du patient. Cette figure a le mérite principal de montrer que le statif de l'invention permet d'obtenir un balayage de l'isocentre O sur tout le corps du patient sans avoir à déplacer la table d'examen. Elle montre également que ce statif permet de dégager la tête du patient ou l'un des côtés longitudinaux à la guise de l'opérateur. Il en résulte un encombrement réduit de l'installation radiologique tout en ayant une plus grande souplesse d'utilisation du statif.

Plus précisément, le statif de la figure 4 diffère de celui de la figure 3 par la manière dont a été réalisée la structure rigide 17. La structure en forme d'arc de cercle 17 a été remplacée par une structure équivalente qui comprend une partie horizontale 24 qui repose sur le sol et se déplace sur ce dernier en rotation en pivotant autour de l'Axe 3. L'extrémité de cette partie horizontale 24, opposée à celle de l'Axe 3, supporte une partie verticale 25 qui pivote autour de l'Axe 4 solidaire de la partie horizontale 24. Cette partie verticale 25 supporte elle-même l'arceau 13 et ses moyens associés de rotation.

L'invention a été décrite en relation avec des exemples particuliers de réalisation mais il est clair qu'elle peut être mise en oeuvre de différentes manières sans sortir de son cadre défini par les revendications ci-après.

## Revendications

1. Statif de radiologie isocentrique comportant une source de rayons X (11) et un détecteur de rayons X (12) portés par un arceau (13) et définissant un axe de chaîne image (RX) passant par un isocentre (O), ledit arceau (13) étant mobile en rotation autour d'un premier axe (Axe 1) perpendiculaire au plan de l'arceau (13) et passant par l'isocentre ainsi qu'autour d'un deuxième axe (Axe 2) dans le plan de l'arceau, ou dans un plan parallèle,perpendiculaire au premier axe et passant également par l'isocentre, ledit arceau étant supporté par une structure rigide (17) qui pivote autour d'un troisième axe (Axe 3) perpendiculaire aux premier et deuxième axes et passant par l'isocentre (O), caractérisé en ce qu'il comprend des moyens de rotation pour faire tourner l'isocentre autour d'un quatrième axe (Axe 4) perpendiculaire au deuxième axe et parallèle au troisième axe.

2. Statif de radiologie isocentrique selon la revendication 1, caractérisé en ce que la structure rigide (17)a la forme d'un arc de cercle dont une extrémité est fixée pivotante au sol et dont l'autre extrémité supporte l'arceau (13) et ses moyens associés de rotation autour des premier et deuxième axes ainsi que les moyens de rotation de l'isocentre (O) autour du quatrième axe.

3. Statif de radiologie selon la revendication 2, caractérisé en ce que la structure rigide (17) comprend une première partie horizontale (24) qui repose et se déplace sur le sol en pivotant autour de l'Axe 3 et une deuxième partie verticale (25) qui supporte l'arceau (13) et ses moyens associés de rotation autour des premier et deuxième axes ainsi que les moyens de rotation de l'isocentre O autour du quatrième axe (Axe 4).

4. Statif de radiologie selon l'une quelconque des revendications précédentes, caractérisé en ce que des moyens sont prévus pour effectuer des déplacements angulaires coordonnés autour des troisième (Axe 3) et quatrième axes (Axe 4) de manière à déplacer l'isocentre (O) suivant une courbe déterminée dans le plan horizontal passant par l'isocentre (O).

5. Statif de radiologie suivant la revendication 4 carractérisé en ce que la courbe de déplacement de l'isocentre est une droite sécante au troisième axe qui est obtenue par un déplacement angulaire autour du quatrième axe qui est double de celui autour du troisième axe.

6. Statif de radiologie selon l'une quelconque des re vendications précédentes 1 à 5 caractérisé en ce que le détecteur de rayons X (12) comprend des moyens pour le faire tourner autour de l'axe RX de la chaîne image de manière à compenser la rotation de l'image lors des mouvements angulaires autour des troisième et quatrième axes.

7. Statif de radiologie selon la revendication 6 caractérisé en ce que les moyens de rotation du détecteur de rayons X (12) autour de l'axe RX de la chaîne image sont du type mécanique/ électrique.

8. Statif de radiologie selon la revendication 6 caractérisé en ce que les moyens de rotation de l'image autour de l'axe RX de la chaîne image sont de type électronique.

# FIG_1

# FIG_2

FIG_3

EP 0 345 138 A1

FIG_4

EP 0 345 138 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-4 358 856 (P. M. STIVENDER et al.)<br>* colonne 4, ligne 48 - colonne 5, ligne 17; figures 1,2 *<br>--- | 1 | A 61 B 6/00 |
| Y | EP-A-0 224 886 (SIEMENS AG)<br>* page 3, ligne 26 - page 4, ligne 8, figures 1,2 * | 1 | |
| A | --- | 2-4,6-8 | |
| A | FR-A-2 588 745 (THOMSON-CGR.)<br>* page 4, ligne 1 - page 6, ligne 20; figures 1-5 *<br>--- | 1 | |
| Y | EP-A-0 122 849 (THOMSON-CGR)<br>* page 3, ligne 13 - page 5, ligne 30; figure 1 *<br>----- | 1 | |

DOMAINES TECHNIQUES
RECHERCHES (Int. Cl.4)

A 61 B 6/00

Le présent rapport a été établi pour toutes les revendications

| Lieu de la recherche | Date d'achèvement de la recherche | Examinateur |
|---|---|---|
| BERLIN | 17-08-1989 | WEIHS J.A. |